# EUROPEAN PATENT SPECIFICATION

(11) **EP 1 534 222 B1**
(45) Date of publication and mention of the grant of the patent: **17.10.2007**
(21) Application number: 03793740.6
(22) Date of filing: 20.08.2003
(51) Int. Cl.: A61K 8/18, A61K 8/19, A61K 8/25, A61K 8/41

(54) **HAIR TREATMENT COMPOSITION**
HAARBEHANDLUNGSZUSAMMENSETZUNG
COMPOSITION DE TRAITEMENT CAPILLAIRE

(30) Priority: 04.09.2002 GB 0220580
(43) Date of publication of application: 01.06.2005
(73) Proprietor: UNILEVER PLC, London EC4P 4BQ (GB); UNILEVER N.V., 3013 AL Rotterdam (NL)
(72) Inventor: GILES, Colin, Christopher, David, Bebington, Wirral, Merseyside CH63 3JW (GB); SOUBIRAN, Laurence, Marie, Cecile, Bebington, Wirral, Merseyside CH63 3JW (GB)
(74) Representative: Newbould, Frazer Anthony
(86) International application number: PCT/EP2003/009224
(87) International publication number: WO 2004/022011

(56) References cited:
- EP-A- 0 370 764
- EP-A- 0 726 246
- EP-A- 1 317 917
- WO-A-99/25312
- WO-A-02/087524
- WO-A-03/047541
- US-A- 5 112 603
- US-A- 5 968 491
- MARKLAND W R: "Laponite synthetic clays" CAPLUS, XP002230818
- PATENT ABSTRACTS OF JAPAN vol. 018, no. 001 (C-1148), 6 January 1994 (1994-01-06) & JP 05 246824 A (SHISEIDO CO LTD), 24 September 1993 (1993-09-24) cited in the application

## Description

### TECHNICAL FIELD

The present invention relates to hair treatment compositions comprising aqueous dispersions of water insoluble composite particles and hair treatment ingredients and processes for their preparation. The particles according to the invention provide specific benefits to hair when used in water based hair treatment compositions such as conditioner and shampoo compositions.

### BACKGROUND OF THE INVENTION

A well known problem in the field of hair care is that of how to deposit benefit agents onto the hair from rinse off products like shampoos and conditioners, the problem simply being that a substantial proportion of the benefit agents may be washed away with the rinse water.

Benefit agents include such things as conditioning agents, colourants, feel modifiers, lustrants, sun screens, nutrients, moisturisers, styling agents and medicinal agents (such as germicides, antidandruff and anti-pruritic agents).

Two methods are commonly used to enhance the deposition of benefit agents onto hair.

One method is to use large droplets of oil carrying a benefit agent in a shampoo or conditioner base. This method relies on physical contact between the hair and the droplets, followed by spreading of the oil droplets over the hair surface. This can, however, lead to the hair feeling greasy or heavy, and looking dull and lifeless because of the extensive spreading of the oil and its absorption by the hair.

Another method is to employ a deposition polymer, which is usually a cationic deposition polymer. The use of such polymers is well known in the prior art. In such systems, polymer, benefit agent and any other insoluble materials are deposited onto the hair. This can lead to dulling of the hair, loss of shine and also to a heavy feel for some consumers.

In attempts to overcome these problems in the prior art; it has been considered necessary to balance the benefits of adequate deposition of benefit agents against the avoidance of sensory negatives. A deposition system that fulfils both criteria remains a widely sought after goal in the field, and to this end much research has been carried out.

### PRIOR ART

JP05/246,824 (Shiseido Co Ltd) discloses a hair cosmetic which is a water-in-oil emulsion containing an organically modified clay mineral (prepared by treating a water swelling clay with a nonionic surfactant and a cationic surfactant), a water-soluble high molecular weight substance and a high molecular weight silicone. The benefits claimed are smooth feel, lustre, firmness and flexibility to hair.

US 5786381 (Franklin et al) describes cosmetic compositions containing hydroxy materials, including layered double hydroxides for treating skin, which may be delivered from a shampoo. The hydroxide is combined with an anionic form of a skin benefit agent which may be lactic acid, glycolic acid or alpha-hydroxycaprylic acid.

WO 02 19976 A (Procter and Gamble Co) describes hair conditioning compositions containing a high melting point fatty compound, a particle and a) an amidoamine and an acid or b) a cationic conditioning agent and a low melting point oil. Silicone is an optional ingredient. The particle can be mica, silica, mud or clay. The benefit claimed is improved texture when spread on hands and/or hair.

EP 0726246 A (Rheox International) describes a quaternary ammonium composition comprising a blend of a liquid diluent and a quaternary ammonium compound. One suggested application is as reaction materials in the manufacture of organoclays. It also discloses particles which are formed by admixing clay, quaternary/diluent mixture and water together in conditions to allow the quaternary compound to react with the clay, followed by filtering, washing, drying and grinding.

It has now surprisingly been found that the incorporation of an aqueous dispersion of composite particles which comprise a clay with a net surface charge, a charged organic molecule and a water insoluble hair benefit agent which is immiscible with the charged organic molecule, into a water based hair treatment composition such as a shampoo or conditioner, leads to enhanced deposition of the hair benefit agent onto the hair, thus imparting a high level of benefit, whilst leaving the hair feeling clean, light and airy. A further advantage of the invention is that the efficiency of deposition of the hair benefit agent is increased as less hair benefit agent is washed away with the rinse water.

### DEFINITION OF THE INVENTION

According to a first aspect of the invention, there is provided a hair treatment composition comprising an aqueous dispersion of water insoluble composite particles, the particles comprising:
i) a clay with a net surface charge,
ii) a charged organic molecule comprising at least 6, preferably at least 11, more preferably at least 17 carbon atoms, and
iii) a water insoluble hair benefit agent which is immiscible with the charged organic molecule,
wherein the charge on the charged organic molecule is opposite to the net surface charge of the clay; the hair treatment composition further comprising one or more suitable hair treatment ingredients in a compatible aqueous carrier.

According to a second aspect of the invention, there is provided a process for the preparation of an aqueous dispersion of composite particles, comprising the steps of:
(i) dispersing the water insoluble hair benefit agent and the clay in water to form a dispersed premixture of clay and hair benefit agent,
(ii) combining the premixture with the charged organic molecule.

According to a third aspect of the invention, there is provided a process for the preparation of an aqueous dispersion of composite particles, comprising the steps of:
(i) dispersing the water insoluble hair benefit agent and the charged organic molecule in water to form a dispersed premixture of charged organic molecule and hair benefit agent,
(ii) combining the premixture with the clay.

According to another aspect of the invention, there is provided a process for making a hair treatment composition comprising the steps of:
(i) preparing an aqueous dispersion of composite particles by one of the processes detailed above and
(ii) combining the aqueous dispersion of composite particles with the remaining hair treatment ingredients in a compatible aqueous carrier, without first drying the aqueous dispersion of composite particles.

### DETAILED DESCRIPTION OF THE INVENTION

### Water based hair treatment compositions

Hair treatment compositions include such compositions as shampoo, conditioner, gel, mousse, shower gel, lotion and serum. Such compositions must be water based; by water based is meant that the composition contains at least 20, preferably 35, most preferably 50 percent water by weight of the total composition. Hair treatment compositions are suitably wash off compositions. Particularly preferred composition forms are shampoos and post-wash conditioners.

### The water insoluble composite particles

The water insoluble composite particles essentially comprise a clay, which has a net charge on its layer surface, a charged organic molecule and a hair benefit agent. Without being bound by theory, it may be postulated that the clay and charged organic molecule form a complex within which the hair benefit agent may be trapped or encapsulated.

It is preferred if particles of the invention have a volume-based median particle diameter (D_{0.5}) of from 5 to 450 microns, preferably 10 to 300 microns and most preferably 15 to 200 microns. Particle diameters can be suitably determined using a Malvern Mastersizer (Malvern Instruments, UK) .

### The clay

The clay which is used in the present invention can be any suitable clay material known in the art. In general, the term clay refers to a composition comprising fine particles which have a net electrostatic charge (i.e., positive or negative) on at least one surface. Preferably, the clay has a layered structure comprising sheets of coordinated cations, wherein the cations form tetrahedra or octahedra with close packed oxygens and hydroxyl groups. The surface charge is usually balanced by the presence of charge balancing ions (sometimes called exchangeable ions) which are usually present between the layers of the clay and at the edges of the layers.

A suitable clay for the invention is an anionic clay. The term anionic clays and related terms, as used herein, refer to clays which are negatively charged at their layer surface. In this embodiment, the water insoluble composite particles are formed with a cationic charged organic molecule, which is in addition to and separate from any charge balancing cations which may be present.

The clay may be a natural, synthetic or chemically modified clay. Preferably, the layers of the clay comprise hydrous sheets of octahedrally coordinated aluminium, magnesium or iron, or of tetrahedrally coordinated silicon. The arrangement of octahedrally coordinated cations to coordinating anions in the clay sheets may be dioctahedral or trioctahedral. The charge balancing ions are cations at the layer surfaces and anions at the edges and may be those occurring naturally in the clay or by exchange using ion exchange techniques. The charge balancing cations of the anionic clays employed in the composite particles of the invention will contain cationic counterions such as protons, sodium ions, potassium ions, calcium ions, magnesium ions, and the like.

Preferred anionic clays are 2:1 phyllosilicates, in which the clay layers comprise two tetrahedral sheets to one octahedral sheet. Preferred 2:1 phyllosilicates are smectite clays.

Other suitable clays have a 1:1 phyllosilicate structure, with an assemblage of one tetrahedral to one octahedral; coordinated sheet in the clay layers.

Smectite clays, used in laundry compositions are, for example, disclosed in US Patents Nos 3,862,058, 3,948,790, 3,954,632 and 4,062,647 and in EP-A-299,575 and EP-A-313,146, all in the name of Procter & Gamble Company.

The term smectite clays herein includes both the clays in which aluminium oxide is present in a silicate lattice and the clays in which magnesium oxide is present in a silicate lattice. Typical smectite clay compounds include the compounds having the general formula Al₂(Si₂O₅)₂(OH)₂.nH₂O and the compounds having the general formula Mg₃(Si₂O₅)₂(OH)₂.nH₂O, and derivatives thereof, for example in which a proportion of the aluminium ions are replaced with magnesium ions or a proportion of the magnesium ions are replaced with lithium ions and/or some of the hydroxyl ions are replaced by fluoride ions; the derivatives may comprise a further metal ion to balance the overall charge.

Specific examples of suitable smectite clays include those selected from the classes of the montmorillonites, hectorites, volchonskoites, nontronites, saponites, beidelites and sauconites, particularly those having an alkali or alkaline earth metal ion within the crystal lattice structure. Particularly preferred are hectorites, montmorillonites, nontronites, saponites, beidelites, sauconites and mixtures thereof. More preferably the clay is a synthetic hectorite.

The cation exchange capacity (CEC) of a clay is a well known parameter and may be determined by well established analytical techniques, including by electrodialysis, by exchange with ammonium ion followed by titration or by a methylene blue procedure, all as fully described in Grimshaw, "The Chemistry and Physics of Clays", pp. 264-265, Interscience (1971). It is customary to measure the cation exchange capacity of a clay in terms of milliequivalents per 100 g of dry clay (meq/100 g).

Preferred anionic clays for use in the present invention have a cation exchange capacity of from 0.7 meq/100 g to 150 meq/100 g. Particularly preferred are clays having a cation exchange capacity of from 30 meq/100 g to 100 meq/100 g.

The clays preferably have a volume-based median particle diameter (D_{0.5}) from 0.001 µm to 300 µm, more preferably from 0.01 µm to 100 µm such as from 0.02 µm to 50 µm, even more preferably 0.02 µm to 20 µm. Particle diameters can suitably be determined using a Malvern Mastersizer (Malvern Instruments, UK).

Examples of synthetic hectorites useful in the present invention include those products sold under the trade names Laponite RD, Laponite RDS, Laponite XLG, Laponite XLS, Laponite D, Laponite DF, Laponite DS, Laponite S and Laponite JS (all from Southern Clay products, Texas, USA, a subsidiary of Rockwood).

Examples of montmorillonites (also known as bentonites), which are suitable for use in the present invention include: Gelwhite GP, Gelwhite H, Gelwhite L, Mineral Colloid BP, Mineral Colloid MO, Gelwhite MAS 100 (sc), Gelwhite MAS 101, Gelwhite MAS 102, Gelwhite MAS 103, Bentolite WH, Bentolite L10, Bentolite H, Bentolite L, Permont SX10A, Permont SC20, and Permont HN24 (Southern Clay Products, Texas, USA); Bentone EW and Bentone MA (Dow Corning); Bentonite USP BL 670 and Bentolite H4430 (Whitaker, Clarke & Daniels); Clarit 100 G1 and Clarit 1100 G1 (calcium bentonites from Süd Chemie AG); and Volclay 2 (sodium bentonite from Süd Chemie AG).

Clays may be used in the present invention either singly or in combination with one or more other clays. If a mixture of clays is used, however, it is preferred that either all of the clays are anionic or all of the clays are cationic. However, mixtures of cationic and anionic clays may be used.

Clays may be used as obtained from the supplier and may contain conventional additives such as, for example, disintegrating agents (also known as peptisers) and water of hydration. The clays may be used in their natural state or in a purified or semi-purified form, for example with the removal of impurities.

Another suitable clay for the invention is a cationic clay. The term cationic clays and related terms, as used herein, refers to clays which are themselves cationic in nature i.e., the clays themselves are positively charged at their layer surfaces. In this embodiment, the composite particles are formed with an anionic charged organic molecule which is in addition to and separate from any charge balancing anions which may be present in the clay.

Preferred examples of cationic clays are the natural or synthetic layered double hydroxide (LDH) clays. For instance, layered double hydroxides include compounds of formula

[M₍₁₋ₐ₎Nₐ(OH)₂]^{y+}[X^{x-} ]_{y/x}.zH₂O

where M is selected from divalent metal ions and lithium; N is a trivalent metal ion; X is an anion of charge x-; y+ is the net charge on the mixed metal hydroxide cation; and when M is a divalent metal ion, "a" is a number from 0.17 to 0.5 and y = a; and when M is lithium "a" is a number from 0. 67 to 0.75 and y = (2a-1); and z is a number from 0 to 10.

In these structures, the metal ions occur in layers in which the metal ions are connected together through the OH groups and the anions X, are located in interlayers between layers of metal ions. It is known that X can undergo ion exchange to be replaced by other anions e.g. organic anions. Various applications for these types of hydroxy materials have been described in the scientific literature, notably including their use as chemical catalysts.

A preferred LDH is hydrotalcite, which has the formula:

(Mg)₂(Al)₄(OH)₁₂.(CO₃)₂.4H₂O

Examples of cationic clays in the hydrotalcite category include Pural MG30, Pural MG50, Pural MG70 (from Condea Chemie GmbH, Hamburg, Germany). Hydrotalcites commercially available include Sorbacid EXM 911 and Hycite EM 713 (from Süd Chemie AG). Other examples of layered double hydroxides are Mg₄Al₂(OH)₁₂(SO₄)_{0.8}.xH₂O Magaldrate (Guilini) and Mg₄Al₄(OH)₁₂Cl₂.xH₂O mixed metal hydroxide (Dow Chemicals). Bayerite is also a suitable cationic clay.

Preferred cationic clays for use in the present invention have an ion exchange capacity of from 0.7 meq/100 g to 250 meq/100 g. Particularly preferred are clays having an ion exchange capacity of from 30 meq/100 g to 200 meq/100 g.

In the compositions of the invention, the clay is advantageously present in the form of a dispersion (for example a sol or gel) or a suspension of the clay particles.

### The charged organic molecule

The charged organic molecule bears a charge which is opposite to that of the surface charge of the clay layer, and is derived from an organic ionic compound e.g. a salt. Any suitable charged organic species may be used. The charged organic molecule may be cationic or anionic, depending on the charge type of the clay. Thus a cationic charged organic molecule is used with an anionic clay and an anionic charged organic molecule is used with a cationic clay.

The charged organic molecule comprises an organic molecule with one or more charged substituents. By organic is meant any molecule containing carbon. The charged organic molecule comprises at least 6, preferably at least 11 and more preferably at least 17 carbon atoms. For the avoidance of doubt, the charged organic molecule is not the same as and is separate from the charge balancing ions, which are naturally present in the clay itself.

In order to form the composite particles, the charged organic molecule must be mixed with the clay. Suitably, the charged organic molecule will be added in the form of a neutral salt.

The charged organic molecule preferably contains at least one straight chain or branched alkyl group or carbon based chain, said alkyl group preferably comprises 3 to 22, more preferably 6 to 22 and most preferably 12 to 22 carbon atoms.

Positively charged organic molecules are preferably selected from the cations of cationic surfactants, cationic polymers and fatty amines used in combination with an acid; negatively charged organic molecules are preferably selected from the anions of anionic surfactants, anionic polymers and polyelectrolytes.

The weight ratio of the charged organic molecule to the clay is from 0.05:1 to 20:1, preferably from 0.1:1 to 10:1, and most preferably from 0.2:1 to 5:1. For the avoidance of doubt, the weight of the charged organic molecule mentioned in the above ratio refers to the weight of the charged organic molecule and excludes any counterion in the neutral salt from which the charged molecule may originate.

### Cationic charged organic molecules

The preferred cationic charged organic molecule is the cation of a cationic surfactant.

Cationic surfactants useful in compositions of the invention contain amino or quaternary ammonium hydrophilic moieties which are positively charged in aqueous solutions.

Examples of suitable cationic surfactants are those corresponding to the general formula (I):

[N(R₁)(R₂)(R₃)(R₄)]⁺(X)⁻ (formula I)

in which R₁ and R₂ are independently selected from (a) an aliphatic group of from 1 to 22, preferably 8 to 22, most preferably 16 to 22 carbon atoms, or (b) an aromatic, alkoxy, polyoxyalkylene, alkylamido, hydroxyalkyl, aryl or alkylaryl group having up to 22 carbon atoms;
R₃ and R₄ and are independently selected from (a) an aliphatic group of from 1 to 12, preferably 1 to 6, most preferably 1 to 3 carbon atoms, or (b) an aromatic, alkoxy, polyoxyalkylene, alkylamido, hydroxyalkyl, aryl or alkylaryl group having up to 12, preferably up to 6, most preferably up to 3 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

Preferably, R₁ of formula (I) is independently selected from
(a) an aliphatic group of from 1 to 22, preferably 8 to 22, most preferably 16 to 22 carbon atoms, or
(b) an aromatic, alkoxy, polyoxyalkylene, alkylamido, hydroxyalkyl, aryl or alkylaryl group having up to 22 carbon atoms; and
R₂, R₃ and R₄ and are independently selected from
(a) an aliphatic group of from 1 to 12, preferably 1 to 6, most preferably 1 to 3 carbon atoms, or
(b) an aromatic, alkoxy, polyoxyalkylene, alkylamido, hydroxyalkyl, aryl or alkylaryl group having up to 12, preferably up to 6, most preferably up to 3 carbon atoms; and X is a salt forming anion.

More preferably, R1 of formula (I) is C16 to C22 and R2, R3 and R4 are methyl groups.

Examples of suitable cationic surfactants include quaternary ammonium compounds, particularly quaternary ammonium compounds comprising a single straight or branched alkyl chain and three methyl groups in which said alkyl chain comprises 16 to 22 carbon atoms.

Suitable quaternary ammonium compounds include cetyltrimethylammonium chloride (CTAC), behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, tallowtrimethylammonium chloride, cocotrimethylammonium chloride, PEG-2 oleylammonium chloride, and salts of these, where the chloride is replaced by halogen, (e.g. , bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18.

Further suitable cationic surfactants include didodecyldimethylammonium chloride and dioctadecyldimethylammonium chloride.

Mixtures of any of the foregoing materials may also be suitable.

Preferred quaternary ammonium salts are behenyltrimethylammonium chloride (BTAC) such as Genamin KDMP supplied by Clariant and cetyltrimethylammonium chloride (CTAC), Such as Arquad 16/29 supplied by Akzo Nobel.

Further suitable cationic systems are primary, secondary, and tertiary fatty amines used in combination with an acid to provide the cationic species. The alkyl groups of such amines preferably have from 12 to 22 carbon atoms, and can be substituted or unsubstituted.

Particularly useful are amido substituted tertiary fatty amines, in particular tertiary amines having one C₁₂ to C₂₂ alkyl or alkenyl chain. Such amines, useful herein, include stearamidopropyldimethylamine, stearamidopropyidiethylamine, stearamidoethyldiethylamine, stearamidoethyldimethylamine, palmitamidopropyld imethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachid amidopropyldiethylamine, arachidamidoethyldiethylamine, arachidamidoethyldimethylamine, diethylaminoethylstearamide

Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

As stated previously, these amines are typically used in combination with an acid to provide the cationic species. The preferred acid useful herein includes L-glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal, et al., issued June 23, 1981.

The molar ratio of protonatable amines to H⁺ from the acid is preferably from about 1:0.3 to 1:1.2, and more preferably from about 1:0.5 to about 1:1.1.

Other suitable cationic charged organic molecules for use in the composite particles of the invention are cationic polymers.

These may be homopolymers or be formed from two or more types of monomers. The molecular weight of the polymer will generally be between 5,000 and 10,000,000, typically at least 10,000 and preferably from 100,000 to about 2,000,000 Da. The polymers will have cationic nitrogen containing groups such as quaternary ammonium or protonated amino groups, or a mixture thereof.

Suitable cationic nitrogen polymers are described in the CTFA Cosmetic Ingredient Directory, 3rd edition

The cationic polymers can comprise mixtures of monomer units derived from amine- and/or quaternary ammonium-substituted monomer and/or compatible spacer monomers.

Suitable cationic polymers include, copolymers of 1-vinyl-2-pyrrolidine and 1-vinyl-3-methyl-imidazolium salts (CTFA name Polyquaternium-16); copolymers of 1-vinyl-2-pyrrolidine and dimethylaminoethyl methacrylate, (CTFA name Polyquaternium-11); cationic diallyl quaternary ammonium-containing polymers in particular(CTFA Polyquaternium 6 and Polyquaternium 7, mineral acid salts of amino-alkyl esters of homo-and co-polymers of unsaturated carboxylic acids as described in U.S. Patent 4,009,256; cationic polyacrylamides (as described in WO95/22311).

Cationic polysaccharide polymers suitable for use in compositions of the invention include those with an anhydroglucose residual group, such as a starch or cellulose. Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhodia in their JAGUAR trademark series). Particularly preferred cationic polymers are JAGUAR C13S, JAGUAR C14, JAGUAR C15, JAGUAR C17 and JAGUAR C16 Jaguar CHT and JAGUAR C162.

### Anionic charged organic molecules

The preferred anionic charged organic molecule is the anion of an anionic surfactant.

Examples of suitable anionic surfactants are the alkyl sulphates, alkyl ether sulphates, alkaryl sulphonates, alkanoyl isethionates, alkyl succinates, alkyl sulphosuccinates, alkyl phosphates, alkyl ether phosphates, alkyl carboxylates, alkyl ether carboxylates, alkyl ester carboxylates, N-alkyl sarcosinates, and alpha-olefin sulphonates, especially their sodium, magnesium, ammonium and mono-, di- and triethanolamine salts. The alkyl and acyl groups generally contain from 8 to 22, preferably 12 to 22 carbon atoms, and may be saturated or unsaturated and can contain, in addition to carbon and hydrogen atoms, ether linkages, and other groups such as amino and ester groups. The alkyl ether sulphates, alkyl ether phosphates and alkyl ether carboxylates may contain from 1 to 10 ethylene oxide or propylene oxide units per molecule.

Typical anionic surfactants for use in compositions of the invention include sodium oleyl sulpho succinate, ammonium lauryl sulphosuccinate, ammonium lauryl sulphate, sodium cocoyl isethionate, sodium lauryl isethionate and sodium N-lauryl sarcosinate, sodium lauryl sulphate, sodium lauryl ether sulphate(n)EO, (where n is from 1 to 3), ammonium lauryl sulphate, ammonium lauryl ether sulphate (n) EO, (where n is from 1 to 3), sodium heptadecyl sulphate, sodium and tetra decyl sulphate.

Further suitable anionic charged organic molecules are anionic polymers.

Examples of suitable anionic polymers are polyacrylates, cross-linked polyacrylates (for example Carbopol ETD 2001 supplied by Goodrich), hydrophobically modified polyacrylates (for example Carbopol 2623 supplied by Goodrich), polyalkylacrylates (for example Carbopol. ETD 2020 supplied by Goodrich), polymethacrylates, polymethylvinylether / maleic anhydride (PVM/MA) copolymers (for example the Gantrez AN series supplied by ISP), alkyl esters of PVM/MA copolymers (for example, the Gantrez series supplied by ISP), monoester resins of PVM/MA copolymers (for example, the Gantrez ES series supplied by ISP), polymethylcarboxylates, polysulphonates and polyphosphates. Further suitable polymers are silicone glycol copolymers, such as DC Q2-5220 supplied by Dow Corning.

### Further charged organic molecules

Further charged organic molecules are amphoteric or zwitterionic surfactants.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in composite particles of the invention include lauryl amine oxide, cocodimethyl sulphopropyl betaine and preferably lauryl betaine, cocamidopropyl betaine and sodium cocamphopropionate.

### The water insoluble hair benefit agent

The term "water insoluble hair benefit agent" may be used to mean any suitable hair benefit agent, which is insoluble in water, where "insoluble" preferably means that the material is not soluble in water (distilled or equivalent) at a concentration of 0.1% (w/w), at 25°C.

The term hair benefit agent includes materials which modify the feel of the hair to consumers, also called hair-feel modifying agents. The term also includes solid materials which may provide hair conditioning benefits or scalp benefits such as anti-dandruff action.

In order obtain the enhanced deposition of the invention, the water insoluble hair benefit agent should be immiscible with the charged organic molecule, by which it is meant that the hair benefit agent is has less 0.1% (weight/weight) solubility in the charged organic molecules at a temperature of 100°C and less. The same conditions apply to the solubility of the charged organic molecule in the hair benefit agent. This immiscibility of the two components means that the hair benefit agent is not a diluent or solvent for the charged organic molecule. For the sake of clarity, it is here made explicit that the combinations of diluents and quaternary compounds disclosed in EP 0 726 246 are not included within the scope of this invention.

The hair benefit agent is preferably uncharged.

In a preferred embodiment of the invention, the water insoluble hair benefit agent is a silicone polymer or a mixture of silicones. Examples of silicones which may be used as water insoluble hair benefit agents include the following:

Cylcomethicones and polydiorganosiloxanes, in particular polydimethylsiloxanes which have the CTFA designation dimethicone. A suitable commercial cyclomethicone product is DC 245 from Dow Corning. Also suitable for use compositions of the invention are polydimethyl siloxanes having hydroxyl end groups, which have the CTFA designation dimethiconol.

Emulsified silicones may be used in compositions of the invention and will typically have an average silicone droplet diameter in the composition of less than 30, preferably less than 20, more preferably less than 10 microns, ideally from 0.01 to 1 microns. Silicone emulsions having an average silicone droplet diameter of ≤ 0.15 microns are generally termed microemulsions.

Examples of suitable pre-formed emulsions include emulsions DC2-1766, DC2-1784, DC-1785 DC-1786 and microemulsions DC2-1865 and DC2-1870, all available from Dow Corning. These are all emulsions/microemulsions of dimethiconol.

A preferred class of silicones for inclusion in compositions of the invention is amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones) . Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant. Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

Further suitable silicones are silicone elastomers, including crosslinked and non-crosslinked silicone elastomers. One preferred silicone elastomer is DC 9509, supplied by Dow Corning.

Suitable silicone elastomers are disclosed in WO 99/63952, EP 0958 804, US 5 871 761 and EP 0 855 178.

Further preferred water insoluble hair benefit agents are finely divided solids. Especially preferred is polytetrafluoroethylene (PTFE). Further suitable finely divided solids are insoluble particulate metal pyrithiones, particularly zinc pyrithione (hereinafter referred to as ZnPTO). The finely divided solid is preferably used in suspended form. Suspending agents may be anionic, cationic or non-ionic surfactants.

The weight ratio of the clay to the water insoluble hair benefit agent is from 0.05:1 to 1:1, preferably from 0.1:1 to 0.8:1, and most preferably from 0.2:1 to 0.6:1.

### Preparation of the composite particles

A suitable process for the preparation of an aqueous dispersion of composite particles according to the invention, comprises the steps of:
(i) dispersing the water insoluble hair benefit agent and the clay in water to form a dispersed premixture of clay and hair benefit agent,
(ii) combining the premixture with the charged organic molecule.

Another suitable process for the preparation of an aqueous dispersion of composite particles according to the invention comprises the steps of:
(i) dispersing the water insoluble hair benefit agent and the charged organic molecule in water to form a dispersed premixture of charged organic molecule and hair benefit agent,
(ii) combining the premixture with the clay.

Preferably, the clay is added as a dry powder to the aqueous premixture of hair benefit agent and charged organic molecule.

A suitable process for making a hair treatment compositions according to the invention comprises the steps of:
(i) preparing an aqueous dispersion of composite particles by one of the processes described above and
(ii) combining the aqueous dispersion of composite particles with the remaining hair treatment ingredients in a compatible aqueous carrier without first drying the aqueous dispersion of composite particles.

It is highly preferred that the composite particles remain as an aqueous dispersion, without being dried, prior to incorporation into a hair treatment composition. This preserves the structure of the particles.

### Hair treatment compositions

The aqueous dispersion of composite particles may be added to one or more suitable hair treatment ingredients to form a hair treatment composition. Compositions in accordance with the invention are preferably formulated as products for the treatment of hair and/or scalp and subsequent rinsing.

Examples of hair treatment compositions that may be formed according to the present invention are a rinse off conditioner comprising a hair conditioning agent and a shampoo comprising a cleansing surfactant. By rinse off conditioner is meant a rinse off hair conditioner product, which is not a shampoo.

The weight of composite particles in the compositions of the invention is preferably from 0.01 to 20, more preferably 0.05 to 10 and most preferably 0.05 to 5 percent by weight of the total weight of the composition.

In a further aspect, the invention provides a method for treating hair and/or scalp, which comprises applying to the hair and/or scalp a hair treatment composition comprising an aqueous dispersion of the water insoluble composite particles, followed by rinsing with water.

In a further aspect, the invention provides a method for depositing hair benefit agents onto hair and/or scalp, which comprises applying to the hair and/or scalp a hair treatment composition comprising an aqueous dispersion of the water insoluble composite particles, followed by rinsing with water.

In another aspect, the invention provides a use of a hair treatment compositions according to the invention to deposit water insoluble hair benefit agents onto hair and/or scalp.

### Hair Treatment Compositions

Compositions in accordance with the invention are preferably formulated as products for the treatment of hair and subsequent rinsing.

### Shampoo Compositions

Shampoo compositions preferably comprise one or more cleansing surfactants which are cosmetically acceptable and suitable for topical application to the hair. Further surfactants may be present as emulsifiers.

Suitable cleansing surfactants, are selected from anionic, amphoteric and zwitterionic surfactants, and mixtures thereof. The cleansing surfactant may be the same surfactant as the emulsifier, or may be different.

### Anionic Cleansing Surfactant

Shampoo compositions according to the invention will typically comprise one or more anionic cleansing surfactants which are cosmetically acceptable and suitable for topical application to the hair. These anionic surfactants may be the same as, but are additional to and separate from, the anionic surfactants which may be used to form part of the composite particles.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, alkyl ester carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl sulpho succinate, ammonium lauryl sulphosuccinate, ammonium lauryl sulphate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n is from 1 to 3), ammonium lauryl sulphate and ammonium lauryl ether sulphate (n) EO, (where n is from 1 to 3).

The total weight of anionic cleansing surfactant in shampoo compositions of the invention is generally from 5 to 30, preferably from 6 to 20, more preferably from 8 to 16 percent by weight of the composition, excluding any anionic surfactant which may be present in the composite particles.

### Co-surfactant

The shampoo composition can optionally include co-surfactants, preferably an amphoteric or zwitterionic surfactant, which can be included in an amount ranging from 0 to about 8, preferably from 1 to 4 percent by weight of the composition. These may be the same as, but are additional to and separate from, the amphoteric or zwitterionic surfactants which may be used to form part of the composite particles.

Examples of amphoteric and zwitterionic surfactants include, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred co-surfactant is a nonionic surfactant; which can be included in an amount ranging from 0 to 8, preferably from 2 to 5 percent, by weight of the compostion.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO-(G)ₙ

wherein R is a branched or straight chain C₅ to C₂₀ alkylor alkenyl group, G is a saccharide group and n is from 1 to 10. Other sugar-derived nonionic surfactants which can be included in shampoo compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The shampoo composition can also optionally include one or more cationic co-surfactants included in an amount ranging from 0.01 to 10, more preferably from 0.05 to 5, most preferably from 0.05 to 2 percent by weight of the composition. Useful cationic surfactants are described hereinbelow in relation to conditioner compositions.

The total weight of surfactant (including any co-surfactant, and/or any emulsifier, but excluding any surfactant which may be present in the composite particles) in shampoo compositions of the invention is generally from 5 to 50, preferably from 5 to 30, more preferably from 10 to 25 percent by weight of the composition.

### Cationic Polymer

The shampoo composition can optionally include cationic polymer(s), which are separate from the cationic polymers which may be used to form part of the composite particles and which are described above. Suitable cationic polymers for use in shampoo compositions of the invention are the same as those described above.

The cationic polymer will generally be present in compositions of the invention at levels of from 0.01 to 5, preferably from 0.05 to 1, more preferably from 0.08 to 0.5 percent by weight of the composition, excluding any cationic polymer which may be present in the composite particles.

### Conditioner Compositions

### Conditioning Surfactant

Conditioner compositions usually comprise one or more conditioning surfactants which are cosmetically acceptable and suitable for topical application to the hair.

Suitable conditioning surfactants are selected from cationic surfactants, used singly or in admixture. These cationic surfactants may be the same as, but are in addition to and separate from, the cationic surfactants which may be used to form part of the composite particles and which are described above.

Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

[N(R₁)(R₂)(R₃)(R₄)]⁺(X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

The most preferred cationic surfactants for conditioner compositions of the present invention are monoalkyl quaternary ammonium compounds in which the alkyl chain length is C16 to C22.

Examples of suitable cationic surfactants include quaternary ammonium compounds, particularly trimethyl quaternary compounds.

Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these, where the chloride is replaced by halogen, (e.g. , bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. Particularly useful quaternary ammonium cationic surfactants for use in hair conditioners of the invention are cetyltrimethylammonium chloride, available commercially, for example as GENAMIN CTAC, ex Hoechst Celanese and Arquad 16/29 supplied by Akzo Nobel, and behenyltrimethylammonium chloride (BTAC) such as Genamin KDM-P supplied by Clariant. These may be the same as but are in addition to and separate from any quaternary ammonium compounds that may be used as charged organic molecule.

Further suitable cationic systems are primary, secondary, and tertiary fatty amines used in combination with an acid to provide the cationic species. These are the same as but are in addition to and separate from any such amines and acids that may be used as charged organic molecule. The alkyl groups of such amines preferably have from 12 to 22 carbon atoms, and can be substituted or unsubstituted.

Particularly useful are amido substituted tertiary fatty amines, in particular tertiary amines having one C₁₂ to C₂₂ alkyl or alkenyl chain. Such amines, useful herein, include stearamidopropyIdimethylamine, stearamidopropyidiethylamine, stearamidoethyldiethylamine, stearamidoethyldimethylamine, palmitamidopropyld imethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachid amidopropyldiethylamine, arachidamidoethyldiethylamine, arachidamidoethyldimethylamine, diethylaminoethylstearamide.

Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

As stated previously, these amines are typically used in combination with an acid to provide the cationic species. The preferred acid useful herein includes L- glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal, et al., issued June 23, 1981.

The molar ratio of protonatable amines to H⁺ from the acid is preferably from about 1:0.3 to 1:1.2, and more preferably from about 1:0.5 to about 1:1.1.

In the conditioners of the invention, the level of cationic surfactant is preferably from 0.01 to 10, more preferably 0.05 to 5, most preferably 0.1 to 2 percent-by weight of the total composition, excluding any cationic surfactant which may be present in the composite particles.

### Fatty Materials

Conditioner compositions of the invention preferably additionally comprise fatty materials. The combined use of fatty materials and cationic surfactants in conditioning compositions is believed to be especially advantageous, because this leads to the formation of a structured lamellar or liquid crystal phase, in which the cationic surfactant is dispersed.

By "fatty material" is meant a fatty alcohol, an alkoxylated fatty alcohol, a fatty acid or a mixture thereof.

Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty material in conditioners of the invention is suitably from 0.01 to 15, preferably from 0.1 to 10, and more preferably from 0.1 to 5 percent by weight of the total composition. The weight ratio of cationic surfactant to fatty alcohol is suitably from 10:1 to 1:10, preferably from 4:1 to 1:8, optimally from 1:1 to 1:7, for example 1:3.

### Optional Ingredients

### Suspending Agents

In a preferred embodiment, the hair treatment composition, especially if it is a shampoo composition, further comprises from 0.1 to 5 percent of a suspending agent, by weight of the total composition. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Compositions of this invention may contain any other ingredient normally used in hair treatment formulations, including further silicone or non-silicone hair conditioning oils. These other ingredients may include viscosity modifiers, preservatives, colouring agents, polyols such as glycerine and polypropylene glycol, chelating agents such as EDTA, antioxidants, fragrances, antimicrobials and sunscreens. Each of these ingredients will be present in an amount effective to accomplish its purpose. Generally these optional ingredients are included individually at a level of up to 5% by weight of the total composition.

### EXAMPLES

The invention will now be further illustrated by the following, non-limiting Examples, in which parts and percentages are by weight unless otherwise stated.

The materials used in the examples were as follows:

**Table A**

| **Chemical** | **Active level** | **Trade Name** | **Supplier** |
|---|---|---|---|
| Silicone emulsion | 50 % | DC1784 | Dow Corning |
| (dimethicone) | 60 % | DC1766 | |
| Silicone elastomer | 60 % | DC9509 | Dow Corning |
| Behenyltrimethyl ammonium chloride (BTAC) | 85 % | *Genamin KDMP | Clariant |
| Cetearyl alcohol | 100 % | *Laurex CS | Ellis & Everard |
| Methyl paraben | 100 % | *Nipagen M | Chemlink Specialities |
| Synthetic clay (hectorite) | 100 % | *Laponite XLS | Laporte |
| Sodium Lauryl Ether Sulphate (SLES) | 28 % | Empicol ESB 28 | Albright & Wilson |
| Cocoamidopropyl Betaine (CAPB) | 30 % | Tegobetaine CK | Goldschmidt |

| | | | |
|---|---|---|---|
| * Trade Mark | | | |

In the following examples, references to stirring at high, medium and low shear rates can be taken as meaning as follows:
High shear: a speed setting of 9-10 on a Silverson mixer, such as a Silverson L4R, ex-Silverson;
Medium shear: a speed setting of 3-4 on a Silverson mixer; Low shear: using a Heidolph mixer, such as a Heidolph RZR 2020, ex-Heidolph Instruments.

### Example 1 - Preparation of composite particles

Composite particles P1, P2, P3 and P4, containing a silicone hair benefit agent, were prepared from the raw materials shown in Table 1. P4 contained a mixture of two types of silicone hair benefit agent. Weight percents are of the raw material as 100% active and based on the weight of the total composition.

**Table 1**

| **Raw material** | **weight %** | | | |
|---|---|---|---|---|
| | **P1** | **P2** | **P3** | **P4** |
| silicone ex. DC1784 | 5.0 | 5.0 | - | - |
| silicone ex. DC1766 | - | - | - | 10.0 |
| silicone elastomer ex. DC9509 | - | - | 10.0 | 10.0 |
| BTAC | 2.0 | 1.0 | 1.0 | 2.0 |
| synthetic clay (hectorite) | 4.0 | 2.0 | 2.0 | 4.0 |
| Water | to100 | to100 | to100 | to100 |

The silicone (emulsion and/or elastomer) was dispersed into the demineralised water (with stirring at medium shear for 3 minutes). The dispersion was then heated to 60 °C. The behenyl trimethyl ammonium chloride (BTAC) was added and mixed with stirring for 30 minutes at medium shear until it had completely dissolved. The resulting blend was allowed to cool to 30 - 35 °C. The clay was then added into the cooled blend in powder form and stirred under medium shear for 15 minutes. The product obtained was a suspension of white particles in water.

The volume based median particle diameter (D_{0.5}) of the white particles (P3) was 57 microns, as determined using a Malvern Mastersizer (Malvern Instruments, UK).

### Examples 2 to 5 - Hair conditioner compositions containing composite particles

Hair conditioner base was prepared from the raw materials shown in Table 2. Weight percents are of the raw material as 100% active and based on the weight of the total composition.

**Table 2**

| **Raw material** | **weight %** |
|---|---|
| BTAC | 2.0 |
| Cetearyl alcohol | 4.0 |
| methyl paraben | 0.2 |
| Water | to 100 |

The base was prepared by heating the water to above 85 °C. The methyl paraben was added with stirring at a high shear rate until it dissolved. The BTAC and Cetearyl alcohol were melted together and added to the aqueous methyl paraben solution with stirring under high shear for 3 minutes and was then allowed to cool to room temp.

The suspensions of composite particles as obtained in Example 1 (P1, P2, P3 or P4 respectively) were well shaken before being dosed into the base shown in Table 2 with medium shear. The suspension was added to the base, in an amount of 10 percent by weight of the total composition (suspension plus base), with stirring for 10 minutes. Example 2 was thus prepared from composite particles P1, Example 3 from P2, Example 4 from P3 and Example 5 from P4 to give the compositions shown in Table 3 below. Comparative examples A (silicone DC1784 in water), B (silicone DC1784 in conditioner base) and C (DC1766 in conditioner base) are also shown.

**Table 3**

| Raw materials used in the preparation of Examples 2 to 5 and Comparative Examples A to C. Weight percents are of the raw material as supplied and based on the weight of the total composition. | | | | | | | |
|---|---|---|---|---|---|---|---|
| **Example** | **2** | **3** | **4** | **5** | **A** | **B** | **C** |
| **In Conditioner Base** | | | | | | | |
| BTAC | 1.8 | 1.8 | 1.8 | 1.8 | - | 1.8 | 1.8 |
| Cetearyl alcohol | 3.6 | 3.6 | 3.6 | 3.6 | - | 3.6 | 3.6 |
| methyl paraben | 0.18 | 0.18 | 0.18 | 0.18 | - | 0.18 | 0.18 |
| | In Composite Particles of example 1 | | | | Added directly to base | | |
| | (P1) | (P2) | (P3) | (P4) | | | |
| BTAC | 0.2 | 0.1 | 0.1 | 0.2 | - | - | - |
| synthetic clay (hectorite) | 0.4 | 0.2 | 0.2 | 0.4 | - | - | - |
| silicone emulsion DC1784 | 0.5 | 0.5 | - | - | 0.5 | 0.5 | - |
| silicone emulsion DC1766 | - | - | - | 1.0 | - | - | 1.0 |
| silicone elastomer DC9509 | - | - | 1.0 | 1.0 | - | - | - |
| water | to 100 | to 100 | to 100 | to 100 | to 100 | to 100 | to 100 |

### Example 6 - Hair shampoo compositions containing composite particles

Hair shampoo base was prepared from the raw materials shown in Table 4. Weight percents are of the raw materials as 100% active and based on the weight of the total composition.

**Table 4**

| **Raw material** | **Weight %** |
|---|---|
| SLES | 14.0 |
| CAPB | 2.0 |
| demineralised water | 82.5 |
| sodium chloride | 1.5 |
| Total | 100 |

The base was prepared by mixing the ingredients together with medium shear.

The suspension of composite particles P1, as obtained in Example 1, was well shaken before being dosed into the base given in Table 4. The suspension was then added to the base, in an amount of 10 percent by weight of the total composition (suspension plus base), with stirring under medium shear for 10 minutes. Example 6 was thus prepared to give the composition as shown in Table 5 below, and Comparative Example D (silicone in shampoo base) was also prepared. Weight percents are of the raw material as supplied and based on the weight of the total composition.

**Table 5**

| **Raw material** | **Example 6** | **Comparative Example D** |
|---|---|---|
| **shampoo base** | | |
| SLES | 12.6 | 12.6 |
| CAPB | 1.8 | 1.8 |
| | In Composite Particles(P1) | Added directly to base |
| silicone emulsion DC1784 | 1.0 | 1.0 |
| BTAC | 0.2 | - |
| synthetic clay | 0.4 | - |
| Water | to100 | to100 |

### Example 7 - Evaluation of silicone deposition onto hair from conditioners

Silicone deposition was evaluated using XRF (X-ray Fluorescence). The XRF instrument used was an energy dispersive X-ray fluorescence spectrometer (ED-XRF), SPECTRO X-LAB 2000. The deposition of silicone onto hair was measured at the K alpha energy 1.744 keV.

A calibration was performed by measuring XRF counts from a series of known levels of silicone dosed onto hair. The deposition of silicone onto the hair from the example compositions was then determined based on this calibration.

The hair used was brown human hair, in switches of 0.25 g in weight and 2 inches in length. 5 switches of hair were used for each evaluation. The hair switches were cleaned prior to treatment as follows: they were wetted with running tap water (approximately 12° FH and approximately 35°) for 10 s. The shampoo base given in Table 4 (0.15 g) was dosed onto the switches (together) and massaged for 30 s before being rinsed for 30 s.

Conditioner composition (0.30 g) was applied to the washed, wet switches and massaged for 1 minute. The conditioner was left on the hair for 1 minute without agitation and rinsed under running tap water for 30 s. The switches were then allowed to dry out before being fixed onto XRF cells and analysed for silicone deposition.

In this way, silicone deposition from the compositions of Examples 3 and 4, and Comparative Examples A and B were measured and are given in Table 6.

**Table 6**

| Si deposition (ppm) onto hair switches from Comparative Examples A and B and Examples 3 and 4 (average of 5 hair switches), measured by XRF. | |
|---|---|
| **Example** | **average (ppm)** |
| A | 105.5 |
| B | 227.9 |
| 3 | 1504.0 |
| 4 | 1430.4 |

It will be seen that deposition of silicone onto hair was dramatically increased from the conditioner compositions in accordance with the invention.

### Example 8: Evaluation of silicone deposition onto hair from shampoo composition

Shampoo composition, Example 6 or Comparative Example D, (0.15 g) was applied to 5 switches, massaged for 30 s and rinsed under running tap water for 30 s. The switches were then allowed to dry out before being fixed onto XRF cells and analysed for silicone deposition using XRF (methodology described in Example 7).

**Table 7: XRF for Si deposition (ppm) air switches from Comparative Example D and Example 6 (average of 5 switches) .**

| **Example** | **average (ppm)** |
|---|---|
| D | 90.875 |
| 6 | 174.64 |

It will be seen that deposition of silicone onto hair was dramatically increased from the shampoo in accordance with the invention.

### Example 9 - Evaluation of hair feel by consumers

The hair used in the evaluation of hair feel was dark brown human hair in switches of approximately 11 inches in length and approximately 10 g in weight. Before use, the switches were balanced with respect to the feel attributes that were to be assessed, namely slippery feel, smooth feel, moisturised feel and powdery/clean feel.

The hair switches were cleaned prior to treatment with the conditioner compositions as follows: they were wetted with running tap water (approximately 12° FH and 35°C) for 10 s. The shampoo base given in Table 4 (0.12 g shampoo per g hair) was dosed onto each switch and massaged for 30 s before being rinsed for 30 s.

Conditioner composition (0.24 g conditioner per g hair) was applied to the washed, wet hair and massaged for 1 minute. The conditioner was left on the hair for 1 minute without agitation and rinsed under running tap water for 30 s. The switches were then allowed to dry. One switch per conditioner composition was used.

In this way, hair switches were treated with Examples 4 and 5 and Comparative Example C before being evaluated for slippery feel, smooth feel, moisturised feel and powdery/clean feel by a panel of 10 consumers. The consumers used a sliding scale from 0 to 100 to evaluate each feel attribute for each composition.

The scores are shown in Table 8 below and are an average of the assessments by the 10 consumers.

**Table 8**

| **Example** | **Feel attribute** | | | |
|---|---|---|---|---|
| | **Slippery** | **Smooth** | **Moisturised** | **Powdery/clean** |
| C | 44.1 | 44.1 | 48.2 | 42.1 |
| 4 | 59.6 | 59.9 | 60.0 | 68.3 |
| 5 | 56.4 | 64.8 | 68.4 | 71.8 |

It will be seen that feel attributes are significantly improved on hair treated with conditioner compositions according to the invention.

## Claims

1. A hair treatment composition comprising an aqueous dispersion of water insoluble composite particles, the particles comprising:
i) a clay with a net surface charge,
ii) a charged organic molecule comprising at least 6 carbon atoms, and
iii) a water insoluble hair.benefit agent which is immiscible with the charged organic molecule and which is a silicone polymer, a mixture of silicones or a finely divided solid,
wherein the charge on the charged organic molecule is opposite to the net surface charge of the clay, the hair treatment composition further comprising one or more suitable hair treatment ingredients in a compatible aqueous carrier,and wherein the weight of composite particles in the composition is from 0.05 to 10 percent by weight of the total weight of the composition.

2. A composition as claimed in Claim 1, wherein the weight ratio of the charged organic molecule to the clay is from 0.05:1 to 20:1.

3. A composition as claimed in claim 1 or claim 2, wherein the volume based median particle diameter of the composite particles is from 5 to 450 microns.

4. A composition as claimed in any preceding claim, wherein the clay has a net negative surface charge and the charged organic molecule has a positive charge.

5. A composition as claimed in claim 4, wherein the clay is a synthetic hectorite.

6. A composition as claimed in claim 4 or claim 5, wherein the charged organic molecule is the cation of an alkyl trimethyl ammonium chloride, wherein the alkyl chain comprises from 12 to 22 carbon atoms.

7. A composition as claimed in claim 4 or claim 5, wherein the charged organic molecule is a cationic polymer.

8. A composition as claimed in any preceding claim, wherein the weight ratio of the clay to the water insoluble hair benefit agent is from 0.05:1 to 1:1.

9. A composition as claimed in any preceding claim, wherein the silicone polymer is a silicone elastomer.

10. A composition as claimed in any preceding claim, which is a rinse off hair conditioner.

## Patentansprüche

1. Haarbehandlungszusammensetzung, umfassend eine wässerige Dispersion aus wasserunlöslichen Verbundteilchen, wobei die Teilchen:
i) einen Ton mit einer Netto-Oberflächenladung,
ii) ein geladenes organisches Molekül, umfassend mindestens 6 Kohlenstoffatome, und
iii) einen wasserunlöslichen Haarwirkstoff, der nicht mit dem geladenen organischen Molekül mischbar ist, und der ein Silikonpolymer, ein Gemisch aus Silikonen oder ein fein zerteilter Feststoff ist, umfassen,
wobei die Ladung an dem geladenen organischen Molekül entgegengesetzt zu der Netto-Oberflächenladung des Tons ist, wobei die Haarbehandlungszusammensetzung ferner einen oder mehrere geeignete Haarbehandlungsinhaltsstoffe in einem kompatiblen wässerigen Träger umfaßt, und wobei das Gewicht der Verbundteilchen in der Zusammensetzung 0,05 bis 10 Gew.-% des Gesamtgewichts der Zusammensetzung beträgt.

2. Zusammensetzung nach Anspruch 1, wobei das Gewichtsverhältnis des geladenen organischen Moleküls zu dem Ton 0,05 : 1 bis 20 : 1 beträgt.

3. Zusammensetzung nach Anspruch 1 oder Anspruch 2, wobei der volumenbasierende mittlere Teilchendurchmesser der Verbundteilchen 5 bis 450 µm beträgt.

4. Zusammensetzung nach einem vorhergehenden Anspruch, wobei der Ton eine negative Netto-Oberflächenladung aufweist und das geladene organische Molekül eine positive Ladung aufweist.

5. Zusammensetzung nach Anspruch 4, wobei der Ton ein synthetischer Hektorit ist.

6. Zusammensetzung nach Anspruch 4 oder Anspruch 5, wobei das geladene organische Molekül das Kation eines Alkyltrimethylammoniumchlorids ist, wobei die Alkylkette 12 bis 22 Kohlenstoffatome umfaßt.

7. Zusammensetzung nach Anspruch 4 oder Anspruch 5, wobei das geladene organische Molekül ein kationisches Polymer ist.

8. Zusammensetzung nach einem vorhergehenden Anspruch, wobei das Gewichtsverhältnis des Tons zu dem wasserunlöslichen Haarwirkstoff 0,05 : 1 bis 1 : 1 beträgt.

9. Zusammensetzung nach einem vorhergehenden Anspruch; wobei das Silikonpolymer ein Silikonelastomer ist.

10. Zusammensetzung nach einem vorhergehenden Anspruch, die eine Haarpflegespülung zum ausspülen ist.

## Revendications

1. Composition de traitement capillaire comprenant une dispersion aqueuse de particules composites non hydrosolubles, lesdites particules comprenant :
i) une argile avant une charge de surface nette,
ii) une molécule organique chargée comprenant au moins 6 atomes de carbone ; et
iii) un agent bénéfique pour les cheveux et non hydrosoluble qui n'est pas miscible avec la molécule organique chargée et qui est un polymère de silicone, un mélange de silicone ou un solide finement divisé,
dans laquelle la charge sur la molécule organique chargée est opposée à la charge de surface nette de l'argile, la composition de traitement capillaire comprenant en outre un ou plusieurs ingrédients de traitement capillaire appropriés dans un support aqueux compatible, et dans laquelle le poids des particules composites dans la composition est de 0,05 à 10 % en poids du poids total de la composition.

2. Composition selon la revendication 1, dans laquelle le rapport pondéral entre la molécule organique chargée et l'argile est de 0,05 :1 à 20 :1

3. Composition selon la revendication 1 ou la revendication 2, dans laquelle le diamètre de particule moyen en volume des particules composites est de 5 à 450 microns.

4. Composition selon l'une quelconque des revendications précédentes, dans laquelle l'argile a une charge de surface nette négative et la molécule organique chargée a une charge positive.

5. Composition selon la revendication 4, dans laquelle l'argile est une hectorite synthétique.

6. Composition selon la revendication 4 ou la revendication 5, dans laquelle la molécule organique chargée est le cation d'un chlorure d'alkyl triméthyl ammonium, dans lequel la chaîne alkyle comprend de 12 à 22 atomes de carbone.

7. Composition selon la revendication 4 ou la revendication 5, dans laquelle la molécule organique chargée est un polymère cationique.

8. Composition selon l'une quelconque, des revendications précédentes, dans laquelle le rapport pondéral entre l'argile et l'agent bénéfique pour les cheveux et non hydrosoluble est de 0,05 : 1 à 1 :1.

9. Composition selon l'une quelconque des revendications précédentes, dans laquelle le polymère de silicone est un élastomère de silicone.

10. Composition selon l'une quelconque des revendications précédentes, qui est un conditionneur pour cheveux à rincer.
